# EUROPEAN PATENT APPLICATION

(11) **EP 4 768 564 A1**
(43) Date of publication of application: **01.07.2026**
(21) Application number: 24872299.3
(22) Date of filing: 25.09.2024
(51) Int. Cl.: C12M 1/00, B01D 29/62, B01D 61/14, B01D 69/06, B01D 69/08, C12M 1/12, C12M 3/06, C12N 1/00, C12P 1/00

(54) **APPARATUS AND METHOD FOR TREATING CELL CULTURE SOLUTION, AND METHOD FOR PRODUCING PRODUCT**

(30) Priority: 27.09.2023 JP 2023166525; 19.02.2024 JP 2024023031
(71) Applicant: FUJIFILM Corporation, Tokyo 106-8620 (JP)
(72) Inventor: HIKICHI, Naomichi, Kanagawa 2588577 (JP); YOSHIDA, Shunichi, Kanagawa 2588577 (JP)
(74) Representative: Hoffmann Eitle
(86) International application number: PCT/JP2024/034230
(87) International publication number: WO 2025/070513

(57) **Abstract**

There is provided a treatment apparatus for a cell culture solution, the treatment apparatus including a bioreactor that accommodates, contains, and stores the cell culture solution, a pipe line in communication with the bioreactor, and a pump unit that feeds the cell culture solution to the pipe line, in which the pump unit includes two or more pump portions that are disposed in the pipe line and that expand and contract in a radial direction of the pipe line, a method for treating a cell culture solution, and a method for producing a product.

## Description

### BACKGROUND OF THE INVENTION

### 1. Field of the Invention

The present disclosure relates to a treatment apparatus for a cell culture solution, a method for treating a cell culture solution, and a method for producing a product.

### 2. Description of the Related Art

For production of a biopharmaceutical or the like, a method for collecting a culture product contained in a cell culture solution by filtration is known. The culture product is recovered from a bioreactor in which the cell culture is performed, via a filtration unit. A pump is used for feeding the cell culture solution from the bioreactor to the filtration unit.

As a pump capable of feeding the cell culture solution, WO2015/039115A discloses the use of a peristaltic pump. WO2012/026978A discloses a filtration system in which feeding of liquid is performed by a diaphragm pump.

### SUMMARY OF THE INVENTION

As the peristaltic pump described in WO2015/039115A, there is also a pump known in the related art that feeds a liquid in a tube by sequentially crushing the tube with a roller. Since the tube is crushed by the roller, damage to the cells is large. On the other hand, by using the diaphragm pump described in WO2012/026978A, the damage to the cells can be reduced. However, the diaphragm pump is not suitable for a case where the amount of the cell culture solution is large because the amount of the liquid fed per unit time is small. A diaphragm pump can cope with a bioreactor having a volume of about 500 L, but in a case where the culture is performed using a bioreactor having a capacity exceeding the volume, it is insufficient because an increased amount of the liquid fed per unit time is required. The same applies to a pump used for feeding a large amount of the cell culture solution in the bioreactor to another tank.

In view of the above circumstances, the object of the technology of the present disclosure is to suppress damage to cells during feeding of a cell culture solution and to enable large-capacity feeding in a treatment apparatus for a cell culture solution, a method for treating a cell culture solution, and a method for producing a product.

A treatment apparatus for a cell culture solution according to the present disclosure includes a bioreactor that accommodates, contains, and stores the cell culture solution, a pipe line in communication with the bioreactor, and a pump unit that feeds the cell culture solution to the pipe line, in which the pump unit includes two or more pump portions that are disposed in the pipe line and that expand and contract in a radial direction of the pipe line.

The pump portion may include a tubular member that has a hollow portion through which the cell culture solution flows, that has a space between an inner wall surface and an outer wall surface, and that has elasticity such that at least the inner wall surface expands and contracts in the radial direction of the pipe line, and the pump unit may include a fluid supply and discharge mechanism that expands the pump portion by supplying a fluid into the space between the inner wall surface and the outer wall surface of the tubular member, and that contracts the pump portion by discharging the fluid from the space.

The pump portion may be a tubular member that has a hollow portion through which the cell culture solution flows, that has a space between an inner wall surface and an outer wall surface, and that has elasticity such that at least the inner wall surface expands and contracts in the radial direction of the pipe line, and the pump unit may include a pressing-force applying mechanism that expands the pump portion by mechanically pressing the inner wall surface of the tubular member toward a radially inner side of the pipe line, and that contracts the pump portion by releasing the pressing, the pump portion being contracted by an elastic force of the tubular member.

A ratio of cross-sectional areas of a flow path of the pump portion in a case where the pump portion is expanded and the flow path of the pump portion in a case where the pump portion is contracted is preferably 0.01 or more.

A spacer that prevents a flow path of the pump portion from being blocked in a case where the pump portion is expanded is preferably disposed in the flow path of the pump portion.

The spacer may be a tubular member formed of a mesh material and having an outer diameter smaller than an inner diameter of the flow path.

A spacer may be provided on a wall surface of the flow path of the pump portion.

The treatment apparatus for a cell culture solution according to the present disclosure may be configured to further include a filtration unit through which the cell culture solution flows in and out and that is disposed in the pipe line and includes a discharge port through which the cell culture solution flows in and out and a filtrate is discharged, in which the cell culture solution is fed from the bioreactor to the filtration unit by expansion and contraction of the pump portions.

In the pipe line, the pump portion may be disposed on each of a downstream side and an upstream side of the filtration unit.

In the pipe line, the filtration unit may be connected to the bioreactor by one conduit.

The treatment apparatus for a cell culture solution according to the present disclosure may be configured to further include a recovery tank that is disposed in the pipe line and recovers the cell culture solution, in which the cell culture solution is fed from the bioreactor to the recovery tank by expansion and contraction of the pump portions.

A method for treating a cell culture solution according to the present disclosure includes a feeding step of disposing, in a pipe line in communication with a bioreactor that accommodates the cell culture solution, two or more pump portions that expand and contract in a radial direction of the pipe line, and feeding the cell culture solution by expanding and contracting the pump portions.

A filtration unit through which the cell culture solution flows in and out and that includes a discharge port through which a filtrate is discharged, may be further provided in the pipe line, the feeding step may be a step of feeding the cell culture solution from the bioreactor to the filtration unit, and the method may include a step of collecting the filtrate from the filtration unit after the feeding step.

The feeding step may include (a) a step of causing the cell culture solution to flow from the bioreactor through the filtration unit for a first period in a first flow direction by sequentially expanding and contracting the two or more pump portions, (b) a step of causing the cell culture solution to flow through the filtration unit for a second period in a second flow direction in which the first flow direction is reversed by sequentially expanding and contracting the two or more pump portions in an order opposite to that in the step (a), (c) a step of causing the cell culture solution to flow through the filtration unit for a third period in the first flow direction in which the second flow direction is reversed by sequentially expanding and contracting the two or more pump portions in the same order as that in the step (a), and (d) a step of repeating the steps (b) and (c) at least twice.

The first period and the second period are each preferably 30 seconds or less.

A method for producing a product according to the present disclosure is a method for producing a product which is a product obtained by culturing cells, the method including: a culturing step of culturing the cells in a bioreactor that accommodates a cell culture solution; a feeding step of disposing, in a pipe line in communication with the bioreactor, two or more pump portions that expand and contract in a radial direction of the pipe line, and feeding the cell culture solution to a filtration unit disposed on the pipe line by expanding and contracting the pump portions; and a collection step of collecting a filtrate containing the product filtered from the cell culture solution by the filtration unit.

According to the treatment apparatus, the method for treating a cell culture solution, and the method for producing a product of the technology of the present disclosure, damage to cells during feeding of a cell culture solution is suppressed, and large-capacity feeding is enabled.

### BRIEF DESCRIPTION OF THE DRAWINGS

FIG. 1 is a diagram showing an overall configuration of a treatment apparatus for a cell culture solution according to an embodiment.
FIG. 2 is a perspective view of a pump portion.
FIG. 3 is an axial cross-sectional view and a radial cross-sectional view of the pump portion in a case where the pump portion is contracted.
FIG. 4 is the axial cross-sectional view and the radial cross-sectional view of the pump portion where the pump portion is expanded.
FIG. 5 is an explanatory diagram of a pump unit of a modification example.
FIG. 6 is an axial cross-sectional view and a radial cross-sectional view of a pump portion of the pump unit of a modification example in a case where the pump portion is contracted.
FIG. 7 is an axial cross-sectional view and a radial cross-sectional view of a pump portion of the pump unit of a modification example in a case where the pump portion is expanded.
FIG. 8A is a perspective view and a radial cross-sectional view of a pump portion including a spacer in a case where the pump portion is contracted, and FIG. 8B is the perspective view and the radial cross-sectional view of the pump portion including the spacer in a case where the pump portion is expanded.
FIG. 9 is a perspective view of a spacer of an example.
FIG. 10A is a perspective view and a radial cross-sectional view of a pump portion including a spacer of a modification example in a case where the pump portion is contracted, and FIG. 10B is the perspective view and the radial cross-sectional view of the pump portion including the spacer of the modification example in a case where the pump portion is expanded.
FIG. 11A is the perspective view and the radial cross-sectional view of a pump portion including a spacer of a modification example in a case where the pump portion is contracted, and FIG. 11B is the perspective view and the radial cross-sectional view of the pump portion including the spacer of the modification example in a case where the pump portion is expanded.
FIG. 12A is the perspective view and the radial cross-sectional view of a pump portion including a spacer of a modification example in a case where the pump portion is contracted, and FIG. 12B is the perspective view and the radial cross-sectional view of the pump portion including the spacer of the modification example in a case where the pump portion is expanded.
FIG. 13A is the axial cross-sectional view and the radial cross-sectional view of a pump portion including a spacer of a modification example in a case where the pump portion is contracted, and FIG. 13B is the axial cross-sectional view and the radial cross-sectional view of the pump portion including the spacer of the modification example in a case where the pump portion is expanded.
FIG. 14 is a diagram showing an overall configuration of a treatment apparatus for a cell culture solution of a modification example.
FIG. 15 is a diagram showing an overall configuration of a treatment apparatus for a cell culture solution according to another embodiment.
FIG. 16 is a diagram showing states before (A) and after (B) connection of pump units, a filtration unit, and a pipe.

### DESCRIPTION OF THE PREFERRED EMBODIMENTS

Hereinafter, the treatment apparatus, the method for treating a cell culture solution, and the method for producing a product according to the present disclosure will be described. However, an embodiment according to the present disclosure is not limited to the following embodiment, and can be implemented by being appropriately modified.

In the present disclosure, the term "process" includes not only an independent process but also a process that cannot be clearly distinguished from other processes, as long as the intended purpose of the process is achieved.

The constituent elements indicated by the same reference numerals in the drawings mean the same constituent elements.

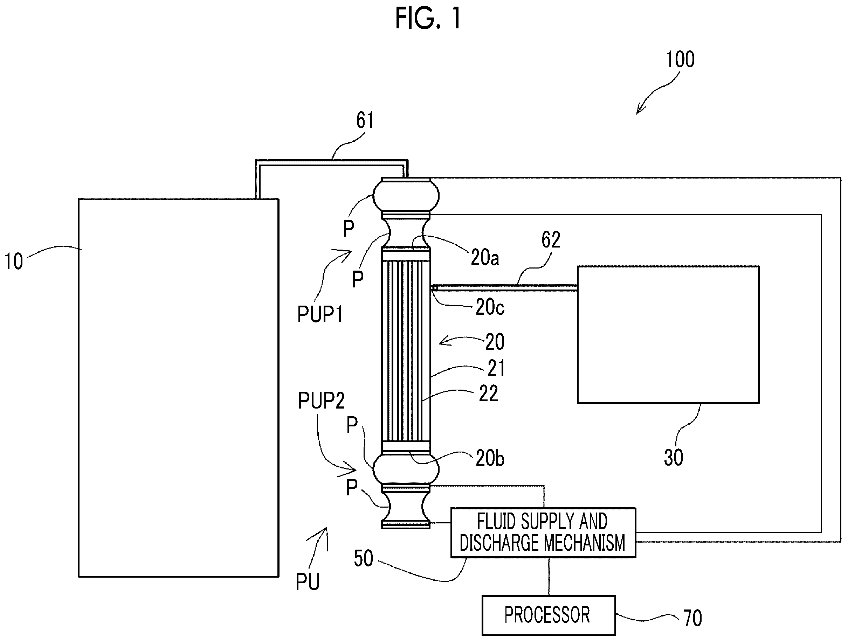

Hereinafter, FIG. 1 is a diagram showing an overall configuration of a treatment apparatus 100 for a cell culture solution according to an embodiment. The treatment apparatus 100 shown in FIG. 1 is an apparatus for producing a product, the product being obtained by culturing cells in a cell culture solution. It is noted that hereafter, the cell culture solution is simply referred to as a "culture solution".

The culture solution contains a culture medium, cells, and a product produced by the cells. The cells have, for example, a diameter of 5 µm or more, and a volume fraction of the cells in the culture solution is, for example, 1% or more. A viscosity µ [Pa·s] of the culture solution changes depending on a type of the culture medium and a concentration of the cells in the culture solution. The viscosity of the culture solution is, for example, 0.5 to 5 mPa·s. Here, the viscosity of the culture solution is a viscosity measured using a vibration-type viscometer after the sampled culture solution is temperature-controlled at 38°C.

The cells are not particularly limited; however, examples thereof include eukaryotic cells such as an animal cell, a plant cell, and yeast, prokaryotic cells such as Bacillus subtilis, and Escherichia coli. Animal cells such as Chinese Hamster Ovary (CHO) cells, Baby Hamster Kidney (BHK)-21 cells, C127 cells, Human Embryonic Kidney (HEK) cells (for example, HEK293), NS0 cells, and SP2/0-Ag14 cells are preferable. CHO cells are more preferable in that a large number of analyses have been performed and genetic engineering methods have been established. Even in a case where the cells do not originally produce the desired product or the production amount is small, the desired product can be efficiently produced, for example, by introducing an expression vector, such as a plasmid, encoding a required protein into the cell.

The product is not particularly limited as long as it is a substance produced by the cells in the culture solution, and examples thereof include substances such as alcohol, enzymes, antibiotics, and proteins. Among these, the product is preferably a protein and a virus, and more preferably an antibody.

In a case where the product is, for example, an antibody, the antibody may be produced by animal cells such as CHO cells. The antibody produced by the animal cell is not particularly limited, but examples thereof include an anti-IL-6 receptor antibody, an anti-IL-6 antibody, an anti-glypican-3 antibody, an anti-CD3 antibody, an anti-CD20 antibody, an anti-GPIIb/IIIa antibody, an anti-TNF antibody, an anti-CD25 antibody, an anti-EGFR antibody, an anti-Her2/neu antibody, an anti-RSV antibody, an anti-CD33 antibody, an anti-CD52 antibody, an anti-IgE antibody, an anti-CD11a antibody, an anti-VEGF antibody, and an anti-VLA4 antibody. The antibody includes not only a monoclonal antibody derived from an animal such as a human being, a mouse, a rat, a hamster, a rabbit, and a monkey but also an artificially modified antibody such as a chimeric antibody, a humanized antibody, and a bispecific antibody.

The product to be intended can be obtained by culturing the cells.

As shown in FIG. 1, the treatment apparatus 100 includes a bioreactor 10, a filtration unit 20, a product recovery unit 30, a pump unit PU including pump portions P, conduits 61 and 62, and a processor 70. It is noted that in the present example, the conduits 61 and 62, the filtration unit 20, and the pump portion P constitute a "pipe line in communication with a bioreactor" in the technology of the present disclosure. The pipe line is a general term for a portion in which the culture solution flows outside the bioreactor 10.

The bioreactor 10 is a culture vessel that accommodates the culture solution and that cultures the cells in the culture solution. The culturing step is performed in the bioreactor 10. The bioreactor 10 preferably has a capacity of more than 500 L. The capacity of the bioreactor 10 is preferably 1,000 L or more and more preferably 2,000 L or more. In addition, the capacity of the bioreactor 10 is preferably 200,000 L or less.

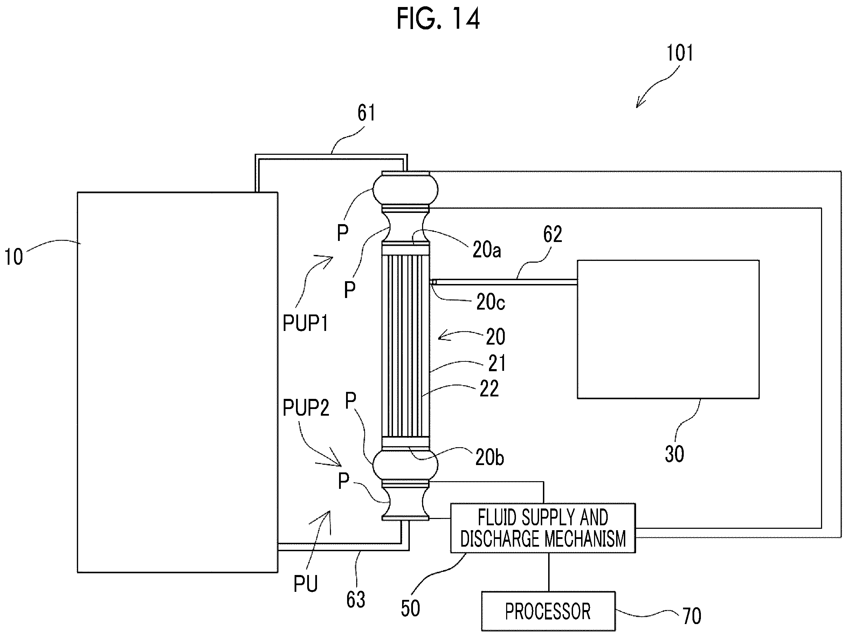

The filtration unit 20 includes, for example, a container 21 and a separation membrane 22 that separates a space in the container 21 into a supply side and a permeation side and that performs a membrane separation treatment on the culture solution fed from the bioreactor 10. The filtration unit 20 includes a first opening 20a and a second opening 20b through which the culture solution flows in and out. The filtration unit 20 includes, on the permeation side, a discharge port 20c connected to the conduit 62 connected to the external product recovery unit 30. In the present example, the culture solution fed from the bioreactor 10 is fed along a membrane surface of the separation membrane 22 while flowing into the container 21 from the first opening 20a and flowing out of the container 21 from the second opening 20b. As a result, in the present example, the membrane separation is performed by a tangential flow method. In the membrane separation treatment by the tangential flow method, a flow in which the culture solution fed from the bioreactor 10 circulates in one direction in parallel along the membrane surface of the separation membrane 22 may be formed, or a flow in which the culture solution reciprocates along the membrane surface of the separation membrane may be formed. It is noted that the treatment apparatus 100 shown in FIG. 1 is a system that forms a flow in which the culture solution reciprocates along the membrane surface of the separation membrane 22 in the filtration unit 20, and a treatment apparatus 101 shown in FIG. 14 described below is a system that can realize both a flow in which the culture solution circulates in one direction along the membrane surface of the separation membrane 22 in the filtration unit 20 and a flow in which the culture solution reciprocates along the membrane surface of the separation membrane 22 in the filtration unit 20.

The separation membrane 22 does not allow the cells in the culture solution to permeate but allows the product produced by the cells to permeate. As the separation membrane 22, a microfiltration membrane, an ultrafiltration membrane, or the like can be used. As the separation membrane 22, a mesh filter, a hollow fiber membrane, or the like can be used.

A permeate containing the product permeates from the supply side to the permeation side of the separation membrane 22 while the culture solution flows along the membrane surface of the separation membrane. The permeate containing the product is discharged from the discharge port 20c to the outside of the container 21 and is recovered by the product recovery unit 30. The product recovery unit 30 includes recovery tank, a purification treatment unit that purifies the product, or the like.

One end of the conduit 61 is connected to the bioreactor 10, and the other end is connected, via a pump portion P described below, to the first opening 20a that is in communication with a supply side of the filtration unit 20. In the present example, the bioreactor 10 and the filtration unit 20 are connected by only one conduit 61.

One end of the conduit 62 is connected to the discharge port 20c of the filtration unit 20, and the other end is connected to the product recovery unit 30.

The pump unit PU feeds the culture solution to the pipe line. In the present example, the pump unit PU includes four pump portions P disposed in the pipe line and a fluid supply and discharge mechanism 50 as a driving unit that drives the pump portions P. Among the four pump portions P, two are disposed on a side of the first opening 20a of the filtration unit 20, and the other two are disposed on a side of the second opening 20b of the filtration unit 20. In a case where the flow from the bioreactor 10 toward the filtration unit 20 is defined as a forward feeding, the first opening 20a is an upstream side, and the second opening 20b is a downstream side. The pump portion P expands and contracts in the radial direction of the pipe line to reduce and enlarge a cross-sectional area of a flow path through which the culture solution flows, and feeds the culture solution from at least the bioreactor 10 to the filtration unit 20. Hereinafter, a portion of the pump unit PU disposed on the side of the first opening 20a is referred to as a first pump unit portion PUP1, and a portion of the pump unit PU disposed on the side of the second opening 20b is referred to as a second pump unit portion PUP2.

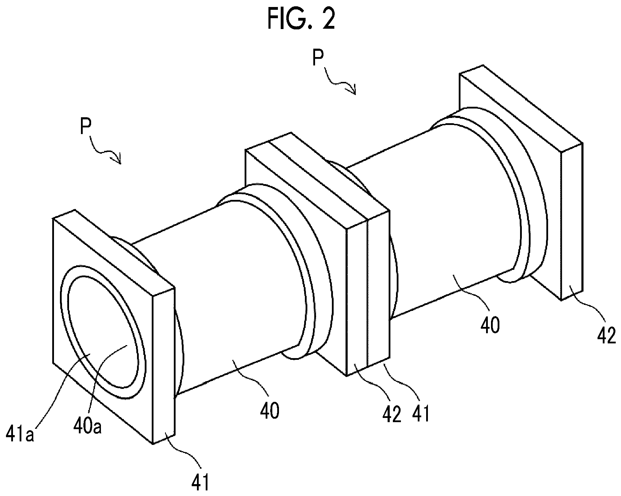

FIG. 2 is a perspective view showing an appearance of the pump portion P. FIG. 2 shows a state in which two pump portions P are connected. The pump portion P includes a main body portion 40 consisting of a tubular member and flanges 41 and 42 provided at both ends of the main body portion 40. The two pump portions P are connected by the flange 42 of one pump portion and the flange 41 of the other pump portion.

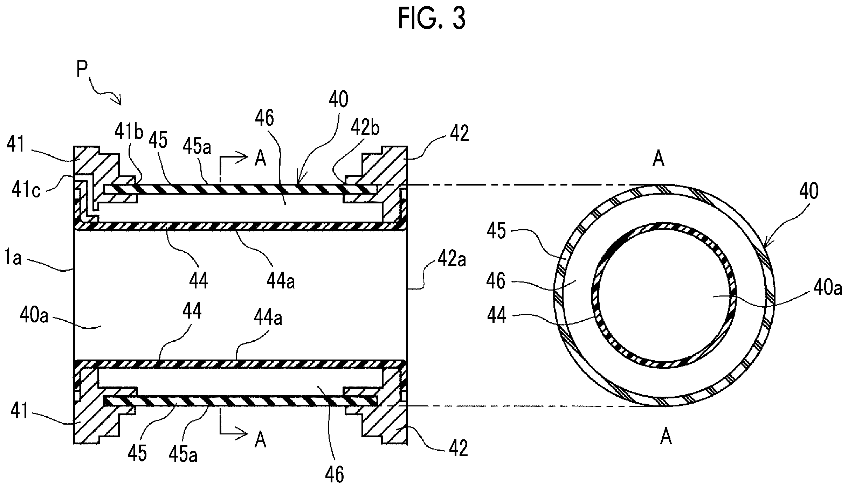

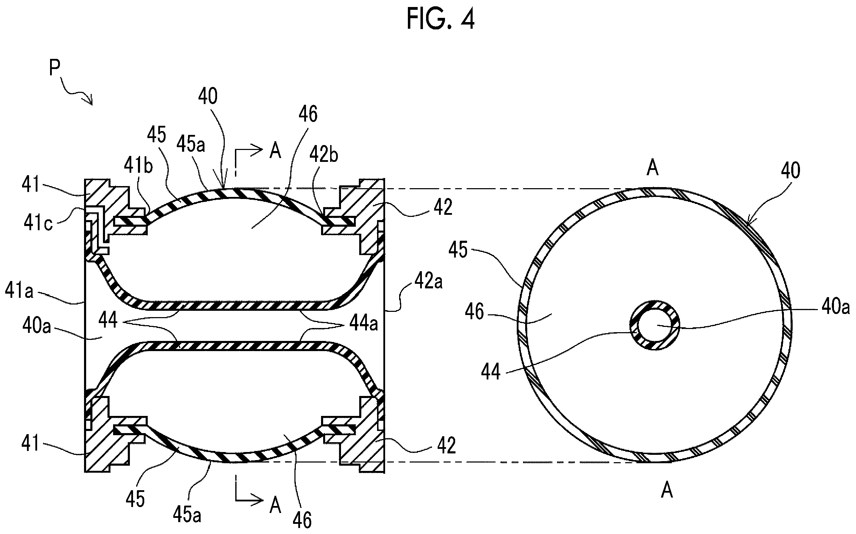

FIG. 3 is a cross-sectional view of the pump portion P along an axial direction and a cross-sectional view of the pump portion P along a radial direction in a case where the pump portion P is contracted. FIG. 4 is a cross-sectional view of the pump portion P along an axial direction and a cross-sectional view of the pump portion P along a radial direction in a case where the pump portion P is expanded. The cross-sectional view along the radial direction (right figure) in FIGS. 3 and 4 is a cross-sectional view taken along the line A-A of the left figure.

As described above, the pump portion P includes the main body portion 40 consisting of a tubular member having a hollow portion 40a through which the culture solution flows, and the flanges 41 and 42 provided at both ends of the main body portion 40. The hollow portion 40a is a flow path through which the culture solution flows, and constitutes a part of the pipe line. The main body portion 40 includes an inner wall member 44 constituting an inner wall surface 44a and an outer wall member 45 constituting an outer wall surface 45a. The main body portion 40 has a space 46 between the inner wall surface 44a and the outer wall surface 45a, that is, between the inner wall member 44 and the outer wall member 45.

The inner wall member 44 is a cylindrical body having elasticity to expand and contract in the radial direction. The inner wall member 44 comes into contact with the culture solution in a case of feeding the culture solution. Therefore, the inner wall member 44 is subjected to a sterilization treatment such as steam sterilization and gamma-ray sterilization. Although details of the sterilization treatment and the like will be described below, it is preferable to use a material that is resistant to the sterilization treatment as the inner wall member 44. Therefore, it is preferable that the inner wall member 44 has, in addition to a high elongation rate (for example, 150% or more) and low leakiness, heat resistance (for example, 120°C or higher) in a case of performing steam sterilization and radiation resistance in a case of performing gamma-ray sterilization. The inner wall member 44 is formed of, for example, rubber such as natural latex rubber and silicone rubber or an elastic material such as an elastomer. More specifically, examples of the material that can be applied to both the steam sterilization and the gamma-ray sterilization include silicone rubber, fluororubber, epichlorohydrin rubber, chloroprene rubber, chlorosulfonated polyethylene (Hypalon), nitrile rubber, acrylic rubber, and a thermoplastic elastomer (for example, a styrene-based thermoplastic elastomer). In addition, examples of the material that can be applied to the gamma-ray sterilization include natural rubber, synthetic natural rubber (for example, isoprene rubber), styrene-butadiene rubber, urethane rubber, and multi-flow addition rubber. In addition, examples of the material that can be applied to the steam sterilization include butyl rubber, ethylene-propylene rubber.

The outer wall member 45 is a cylindrical body that is concentric with the inner wall member 44 and has a larger diameter, and is formed of a metal, a resin, or the like. It is noted that the outer wall member 45 may be formed of the same elastic material as the inner wall member 44. Provided that outer wall member 45 is preferably formed such that the elastic modulus of the outer wall member 45 is larger than the elastic modulus of the inner wall member 44. As an example, in the present embodiment, the outer wall member 45 is formed of a material having a larger elastic modulus than the inner wall member 44.

Both end portions of each of the inner wall member 44 and the outer wall member 45 are fixed to the flange 41 and the flange 42. The flange 41 and the flange 42 each have a flat rectangular shape and have circular center holes 41a and 42a at their center portions. One end of the inner wall member 44 is fixed to the flange 41 to extend around onto a front surface of the flange 41, and the other end is fixed to the flange 42 to extend around onto a front surface of the flange 42. Here, the front surface of the flange 41 and the flange 42 refers to a surface that faces axially outward with respect to the main body portion 40, and the rear surface of the flange 41 and the flange 42 refers to a surface that faces axially inward with respect to the main body portion 40. On the rear surfaces of the flange 41 and the flange 42, grooves 41b and 42b are provided outside center holes 41a and 42a and concentric with the center holes 41a and 42a. The outer wall member 45 is fixed to the flanges 41 and 42 such that one end of the outer wall member 45 is inserted into the groove 41b of the rear surface of the flange 41 and the other end is inserted into the groove 42b of the back surface of the flange 42. It is noted that the front surfaces of the flange 41 and the flange 42 are configured to be connectable to each other.

Both end portions of each of the inner wall member 44 and the outer wall member 45 are fixed to the flanges 41 and 42, and the space 46 between the inner wall surface 44a and the outer wall surface 45a is a closed space. The pump portion P is configured such that, in a case where a fluid is supplied into the space 46, the inner wall member 44 expands in the radial direction of the hollow portion 40a. In the flange 41, a supply and discharge hole 41c in communication with the space 46, for supplying the fluid to the space 46 and discharging the fluid from the space 46. The supply and discharge hole 41c is connected to the fluid supply and discharge mechanism 50. It is noted that the outer wall member 45, the flanges 41 and 42, and the like are also preferably formed of a material that is resistant to the sterilization treatment. Specific examples thereof include stainless steel, polypropylene, polycarbonate, and the like.

The fluid supply and discharge mechanism 50 is a driving unit that drives the pump portion P. The fluid supply and discharge mechanism 50 expands the space 46 by supplying the fluid to the space 46 between the inner wall member 44 and the outer wall member 45 of the pump portion P, and contracts the space 46 by discharging the fluid from the space 46. The expression "expand the space 46" corresponds to "expand the pump portion" in the present disclosure, and the expression "contract the space 46" corresponds to "contract the pump portion" in the present disclosure. In a case where the pump portion P expands, a cross section of the flow path consisting of the hollow portion 40a through which the culture solution flows is reduced, and in a case where the pump portion P contracts, the cross section of the flow path consisting of the hollow portion 40a is increased. That is, the expansion and contraction of the pump portion P mean the reduction and enlargement of the cross section of the flow path.

The fluid may be a gas such as air or a liquid such as water or oil. As an example, the fluid supply and discharge mechanism 50 includes a compressor and supplies and discharges compressed air, as the fluid to and from the space 46 of each pump portion P.

In the treatment apparatus 100, the pump unit PU includes four pump portions P, and two pump portions P are disposed on each of the upstream side and the downstream side of the filtration unit 20, but the pump unit PU may include two or more pump portions P. In a case where the pump unit PU includes only two pump portions P, the pump unit PU may have a configuration in which the two connected pump portions P may be disposed on any one of the upstream side or the downstream side of the filtration unit 20, or one pump portion P may be disposed on the upstream side and the other pump portion P may be disposed on the downstream side of the filtration unit 20. In addition, in a case where the pump unit PU includes three or more pump portions P, three or more pump portions P connected to one another may be disposed only on any one of the upstream side or the downstream side of the filtration unit 20, or the pump portions P may be disposed on each of the upstream side and the downstream side. That is, the pump unit PU may be configured to have only one of the first pump unit portion PUP1 or the second pump unit portion PUP2, or may have a configuration in which each of the first pump unit portion PUP1 and the second pump unit portion PUP2 may include one or two or more pump portions P. By connecting a plurality of pump portions P, the feeding capacity can be increased. It is more preferable that three pump portions P are provided on each of the upstream side and the downstream side of the filtration unit 20. It is preferable that the pump portion P is installed in consideration of a pressure loss in the pump portion P.

The processor 70 controls the fluid supply and discharge mechanism 50 to control the timing of the expansion and contraction of each pump portion P for feeding the culture solution from the bioreactor 10 to the filtration unit 20. The processor 70 can also switch between forward feeding in which the culture solution is fed from the bioreactor 10 to the filtration unit 20 and reverse feeding in which the culture solution is fed from the filtration unit 20 to the bioreactor 10 by controlling the timing of the expansion and contraction of each pump portion P. By shifting the timing of the expansion and contraction of the plurality of pump portions P, the culture solution can be fed. For example, the pump portions P arranged from the upstream side to the downstream side with respect to the flow direction of the culture solution are expanded in the arrangement order such that the cross-sectional area of the hollow portion 40a (that is, the pipe line) is narrowed in order from the pump portion P on the upstream side.

### "Method for treating cell culture solution"

Here, an example of a method for treating a cell culture solution in the treatment apparatus 100 having the above-described configuration will be described.

The method for treating a cell culture solution using the treatment apparatus 100 includes at least a feeding step of feeding the culture solution by expanding and contracting the pump portion P. In the present example, the method for treating a cell culture solution further includes a step (filtrate collection step) of collecting the filtrate from the filtration unit 20. It is noted that since the present treatment method includes a culturing step of culturing the cells in the bioreactor 10 and the filtrate separated from the culture solution by the filtration unit 20 contains the product of the cells, the present treatment method is also an example of a method for producing a product.

The feeding step includes a step of feeding the culture solution from the bioreactor 10 to the filtration unit 20 by sequentially expanding and contracting the four pump portions P of the pump unit PU at a predetermined timing, and, conversely, a step of feeding the culture solution from the filtration unit 20 to the bioreactor 10. In the step of feeding the culture solution from the bioreactor 10 to the filtration unit 20, the culture solution fed from the bioreactor 10 flows inside the filtration unit 20 in a first flow direction in which the culture solution flows into the filtration unit 20 from the first opening 20a toward the second opening 20b. In addition, in the step of feeding the culture solution from the filtration unit 20 to the bioreactor 10, the culture solution flows inside the filtration unit 20 in a second flow direction from the second opening 20b side toward the first opening 20a. That is, the culture solution flows inside the filtration unit 20 to reciprocate along the membrane surface of the separation membrane 22 in the filtration unit 20. While the culture solution flows inside the filtration unit 20 along the membrane surface of the separation membrane 22, the separation membrane 22 allows a filtrate containing the product to permeate to the permeation side. As a result, the cells and the product in the culture solution are separated. Then, in the filtrate collection step, the filtrate is discharged from the discharge port 20c of the filtration unit 20 and is collected by the product recovery unit 30. It is noted that a pump (not shown) may be provided in the conduit 62 that connects the filtration unit 20 and the product recovery unit 30.

As described above, the feeding step of the method for treating a cell culture solution using the present treatment apparatus 100 includes a step of feeding the culture solution from the bioreactor 10 to the filtration unit 20 in the first flow direction and a step of feeding the culture solution from the filtration unit 20 to the bioreactor 10 side in the second flow direction opposite to the first flow direction. More specifically, it is preferable that the feeding step includes (a) a step of causing the culture solution to flow from the bioreactor 10 through the filtration unit 20 for a first period in a first flow direction by sequentially expanding and contracting the plurality of pump portions P, (b) a step of causing the culture solution to flow through the filtration unit 20 for a second period in a second flow direction in which the first flow direction is reversed by sequentially expanding and contracting the plurality of pump portions P in an order opposite to that in the step (a), (c) a step of causing the culture solution to flow through the filtration unit 20 for a third period in the first flow direction in which the second flow direction is reversed by sequentially expanding and contracting the plurality of pump portions P in the same order as that in the step (a), and (d) a step of repeating the steps (b) and (c) at least twice.

In the feeding in the first flow direction in the step (a) and the step (c), that is, in the forward feeding, the culture solution flows into the filtration unit 20 from the first opening 20a, the culture solution flows from the first opening 20a to the second opening 20b in the filtration unit 20, and the culture solution further flows from the second opening 20b to the pump portion P.

In the feeding in the second flow direction in the step (b), that is, in the reverse feeding, the culture solution flows from the pump portion P disposed on the second opening 20b side of the filtration unit 20 into the filtration unit 20 through the second opening 20b. In the filtration unit 20, the culture solution flows from the second opening 20b to the first opening 20a and flows out from the first opening 20a toward the bioreactor 10. While the culture solution flows inside the filtration unit 20 in the first flow direction and the second flow direction, the product is filtered by the separation membrane 22, and the filtrate containing the product moves to the permeation side. In the filtrate collection step, the filtrate is collected by the product recovery unit 30. In the present example, the filtrate collection step is performed in parallel with the feeding step.

The first period in the step (a), the second period in the step (b), and the third period in the step (c) are not particularly limited, but the first period and the second period are each preferably 30 seconds or less and more preferably less than 30 seconds. In addition, the third period is preferably 30 seconds or less and more preferably less than 30 seconds.

In this manner, inside the filtration unit 20, by causing the culture solution to flow in a first flow direction and in a second flow direction and repeatedly reversing the flow direction, it is possible to obtain a flushing effect on the adhering substance such as the clogging substance adhering to the wall surface of the separation membrane 22 and to suppress clogging of the separation membrane 22.

As described above, the treatment apparatus 100 for a cell culture solution according to the present embodiment includes the bioreactor 10, the pipe line in communication with the bioreactor 10, and the pump unit PU, and the pump unit PU includes two or more pump portions P disposed in the pipe line. The pump portion P feeds the culture solution by expanding and contracting in the radial direction of the pipe line. In the tube pump in the related art, since the culture solution is fed while the tube (conduit) is crushed by the rotor to sequentially block the flow path, the shear stress is large and the cells are likely to be damaged. However, in a case where the pump portion P that expands and contracts in the radial direction of the present treatment apparatus 100 is used, the shear stress applied to the culture solution during the feeding can be reduced, and as a result, the damage to the cells can be suppressed. In addition, in the diaphragm pump, it is difficult to increase the flow rate, but according to the pump portion P of the present treatment apparatus 100, the flow rate can also be increased.

The pump portion P of the present embodiment is a tubular member including the hollow portion 40a through which the culture solution flows, having a space 46 between the inner wall surface 44a and the outer wall surface 45a, and having elasticity such that the inner wall surface 44a expands and contracts in the radial direction of the pipe line. Then, the pump unit PU includes the fluid supply and discharge mechanism 50 that expands the pump portion P by supplying the fluid to the space 46 and that contracts the pump portion P by discharging the fluid from the space 46. With the configuration, the culture solution can be fed by a peristaltic motion of expanding or contracting the space 46 to increase or decrease the cross-sectional area of the hollow portion 40a through which the culture solution flows. The amount of the fluid supplied to the space 46 by the fluid supply and discharge mechanism 50 can be adjusted such that the hollow portion 40a that is the flow path is not completely blocked, and the shear stress can be suppressed.

The method for treating a cell culture solution according to the present embodiment includes a feeding step of disposing, in a pipe line in communication with a bioreactor 10 that accommodates the culture solution, two or more pump portions P that expand and contract in a radial direction of the pipe line, and feeding the culture solution by expanding and contracting the pump portions P. By adjusting the expansion and contraction of the pump portion P, the culture solution can be fed without blocking the flow path as in a case where the tube is crushed by the roller in the tube pump in the related art. Therefore, the damage to the cells can be suppressed.

It is noted that as the method for producing a product, perfusion culture further including a supply step of supplying a fresh culture medium to the bioreactor is preferably used. The perfusion culture is a culture method in which a fresh culture medium is added and at the same time the used culture medium is removed. According to the perfusion culture, it is possible to achieve a high cell density exceeding 100 × 10⁶ cells/mL. In addition, an advantage of the perfusion culture is that the culture in which the product to be intended is produced is maintained for a longer period than the batch culture method or the fed-batch culture. A device and a culture method of the perfusion culture are well known in the related art, and are described in WO2018/159847A, WO2019/049843A, WO2019/181234A, WO2019/239780A, WO2020/003833A, WO2020/162125A, WO2021/187008A, WO2022/196710A, and WO2023/054556A, the contents of which are incorporated herein by reference.

In the method for producing a product, the cell density of the culture solution is preferably 30 × 10⁶ cells/mL or more. The cell density of the culture solution is more preferably 50 × 10⁶ cells/mL or more, more preferably 80 × 10⁶ cells/mL or more, more preferably 120 × 10⁶ cells/mL or more, and still more preferably 150 × 10⁶ cells/mL or more. In addition, the cell density of the culture solution is preferably 400 × 10⁶ cells/mL or less and more preferably 250 × 10⁶ cells/mL or less. The cell density can be determined by measuring the number of cells by a general method and dividing the number of cells by the culture solution amount.

In a case where the cell density of the culture solution is high, such as 30 × 10⁶ cells/mL or more, the effect of suppressing the damage to the cells according to the technology of the present disclosure is more effective.

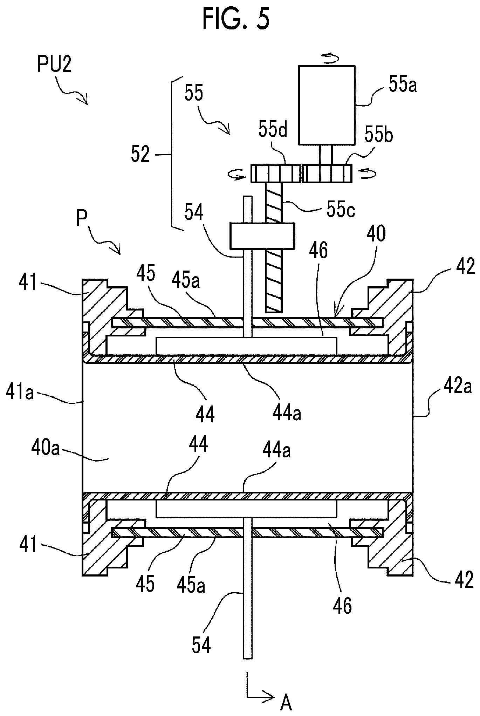

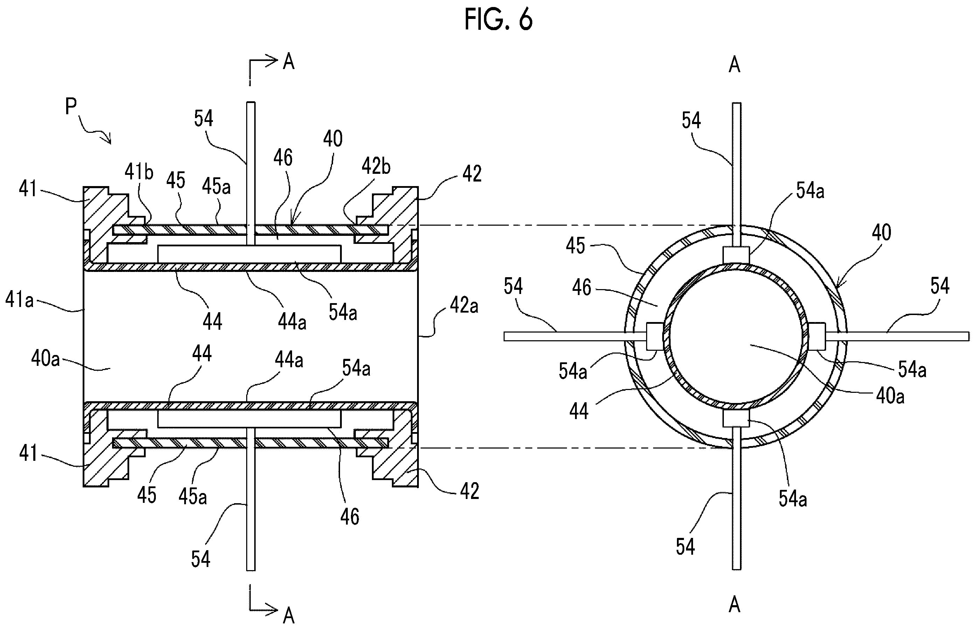

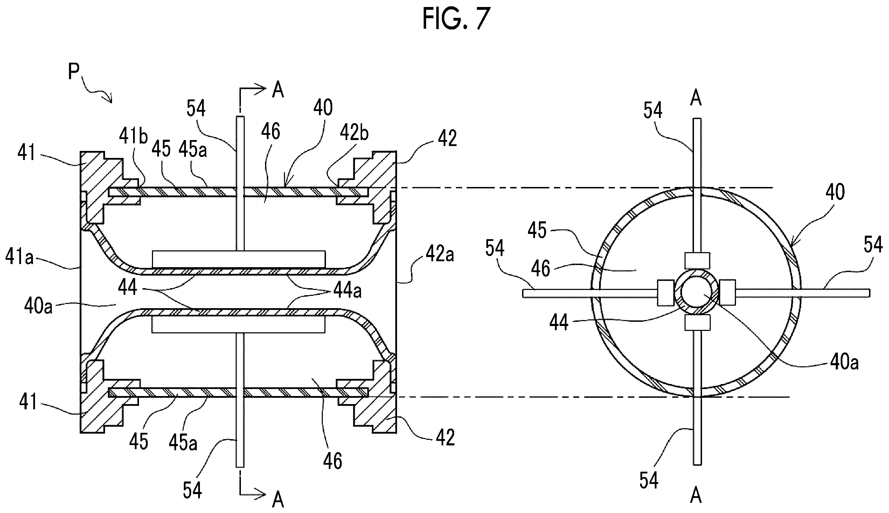

It is noted that the mechanism that expands and contracts the inner wall surface 44a of the pump unit PU is not limited to the fluid supply and discharge mechanism 50 that performs the supply and discharge of the fluid to the space 46 as described above. FIGS. 5 to 7 are explanatory diagrams of a pump unit PU2 of a modification example. FIG. 5 shows one pump portion P and a driving mechanism thereof as a schematic configuration of the pump unit PU2. FIGS. 6 and 7 show a state in which the pump portion P of the pump unit PU2 is contracted and a state in which the pump portion P is expanded. In FIGS. 6 and 7, the left figure is a cross-sectional view along the axial direction of the pump portion P, and the right figure is a cross-sectional view taken along the line A-A of the left figure.

As shown in FIG. 5, the pump unit PU2 of the modification example includes a pressing-force applying mechanism 52 that mechanically presses the inner wall surface 44a toward an inner side of the hollow portion 40a in the radial direction, instead of the fluid supply and discharge mechanism 50 that performs the supply and discharge of the fluid to the space 46. The pressing-force applying mechanism 52 includes a pressing member 54 and a driving unit 55 that drives the pressing member 54. The pressing member 54 has a pressing portion 54a that is disposed in the space 46 between the inner wall member 44 and the outer wall member 45 and that is pressed against the inner wall member 44, and is movable in the radial direction inside the space 46. The driving unit 55 is a moving mechanism that reciprocates the pressing member 54 in the radial direction, and is, for example, a linear actuator including a stepping motor 55a as shown in FIG. 5.

The gear 55b provided at a distal end of the driving unit 55 rotates by rotating a stepping motor 55a, and the driving unit 55 rotates a ball screw 55c via a gear 55d provided at one end of the ball screw 55c. The pressing member 54 is fixed to an attachment portion 55e that moves along the ball screw 55c in conjunction with the rotation of the ball screw 55c, and moves in the axial direction of the ball screw 55c in conjunction with the movement of the attachment portion 55e. The ball screw 55c is disposed to match the axial direction with the radial direction of the hollow portion 40a, and the pressing member 54 is movable in the radial direction of the hollow portion 40a.

As shown in FIGS. 6 and 7, the pressing portion 54a of the pressing member 54 has a shape that extends along the axial direction of the main body portion 40. In the present example, four pressing members 54 are disposed on the outer peripheral surface of the inner wall member 44 at 90° intervals. Each of the four pressing members 54 is configured to be movable in the radial direction by the driving unit 55. As shown in FIG. 7, the four pressing members 54 press the inner wall member 44 inward, thereby expanding the inner wall member 44, and as a result, the pump portion P is expanded. By returning the pressing member 54 to the position shown in FIG. 6, pressing on the inner wall member 44 is released, the inner wall member 44 is contracted, and as a result, the pump portion P is contracted. The driving unit 55 is controlled by the processor 70.

As in the pump unit PU2 of the modification example, the culture solution can be fed by a peristaltic motion of expanding or contracting the space 46 to increase or decrease the cross-sectional area of the hollow portion 40a through which the culture solution flows, by including the pressing-force applying mechanism 52 that mechanically presses the inner wall surface 44a toward the inner side of the hollow portion 40a in the radial direction. By controlling the amount of movement of the pressing member 54 in the radial direction by the pressing-force applying mechanism 52, the hollow portion 40a that is the flow path can be adjusted not to be completely blocked, and the shear stress can be suppressed.

In a case of feeding the culture solution by the pump unit PU of the treatment apparatus 100 of the above-described embodiment or the pump unit PU2 of the modification example, a ratio S2/S1 of a cross-sectional area S2 of the hollow portion 40a in a case where the pump portion P shown in FIG. 4 or FIG. 6 is expanded to a cross-sectional area S1 of the hollow portion 40a in a case where the pump portion P shown in FIG. 3 or FIG. 5 is contracted is preferably 0.01 or more. The cross-sectional areas S1 and S2 are cross-sectional areas at substantially central positions of the main body portion 40 in the axial direction. By preventing the hollow portion 40a from being completely blocked in a case of expansion, the damage to the cells can be suppressed.

It is noted that as described above, by controlling the expansion of the pump portion P by the fluid supply and discharge mechanism 50 or the pressing-force applying mechanism 52, the damage to the culture solution and the cells can be suppressed, but to more reliably prevent the flow path consisting of the hollow portion 40a of the pump portion P from being blocked, it is preferable to provide a spacer 80 in the hollow portion 40a.

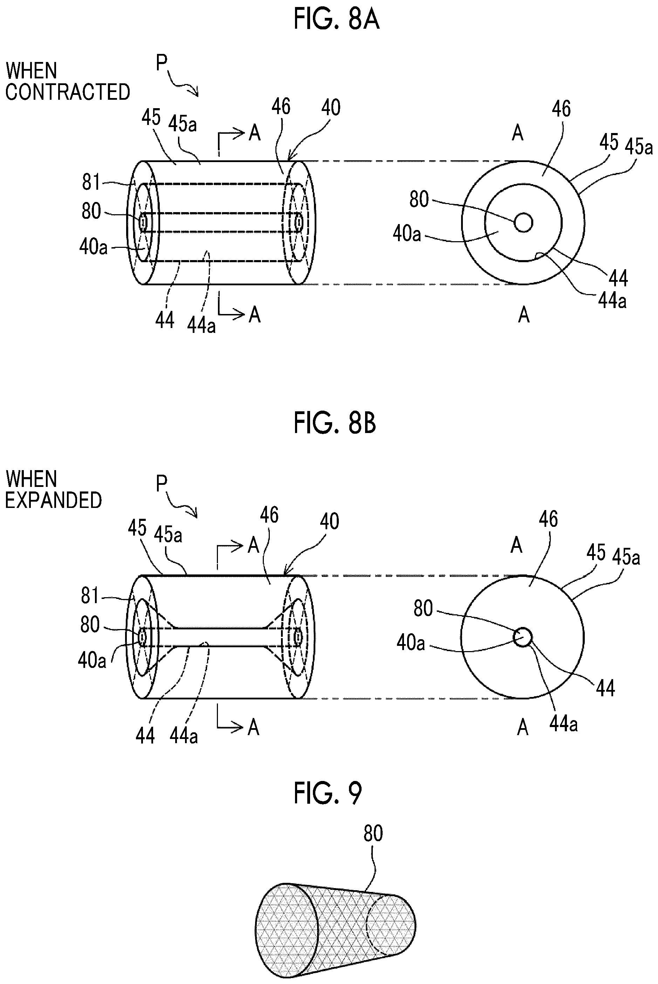

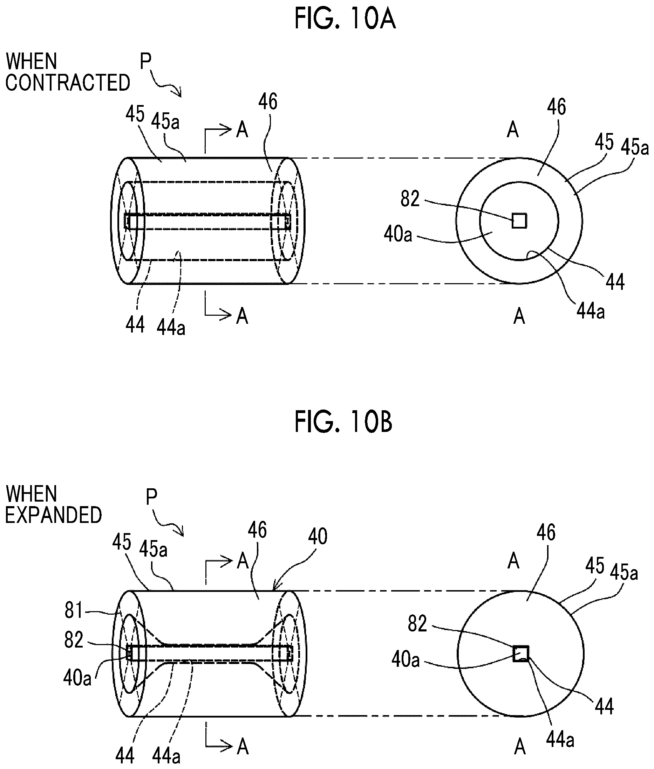

FIG. 8A shows a state in which the pump portion P including the spacer 80 is contracted, and FIG. 8B shows a state in which the pump portion P is expanded. FIG. 9 is a perspective view of the spacer 80. In FIGS. 8A and 8B, the left figure is a perspective view of the pump portion P, and the right figure is a cross-sectional view taken along the line A-A in the left figure. It is noted that in FIGS. 8A and 8B, the thicknesses of the inner wall member 44 and the outer wall member 45 and the flanges 41 and 42 at both ends of the main body portion 40 are not shown. The same applies to FIGS. 10A to 12A and FIGS. 10B to 12B described below.

As shown in FIG. 9, the spacer 80 is a cylindrical member formed of a mesh material. Then, the spacer 80 has an outer diameter smaller than an inner diameter of the hollow portion 40a. The spacer 80 of the present example has a length substantially equal to a length of the main body portion 40. As shown in FIG. 8A, the spacer 80 is disposed at substantially the center of the hollow portion 40a of the main body portion 40. As shown in FIG. 8A, the spacer 80 is fixed by a fixing portion 81 shown by a broken line at an end portion of the main body portion 40. As shown in FIG. 8B, in a case where the pump portion P is expanded, that is, in a case where the space 46 is expanded, the expansion of the inner wall member 44 is restricted by the spacer 80 in a case where the inner wall member 44 is expanded toward the radial center of the hollow portion 40a. The blocking of the hollow portion 40a can be reliably prevented by the spacer 80.

The spacer 80 is a cylindrical member, but the spacer in the technology of the present disclosure is not limited to the cylindrical shape. As shown in FIGS. 10A and 10B, the spacer 82 may be a spacer consisting of a tubular member having a quadrangular prism-shaped external shape. In addition, although not shown, the spacer may be a tubular member having a triangular prism-shaped external shape or a tubular member having a polygonal prism-shaped external shape of a pentagonal prism or more.

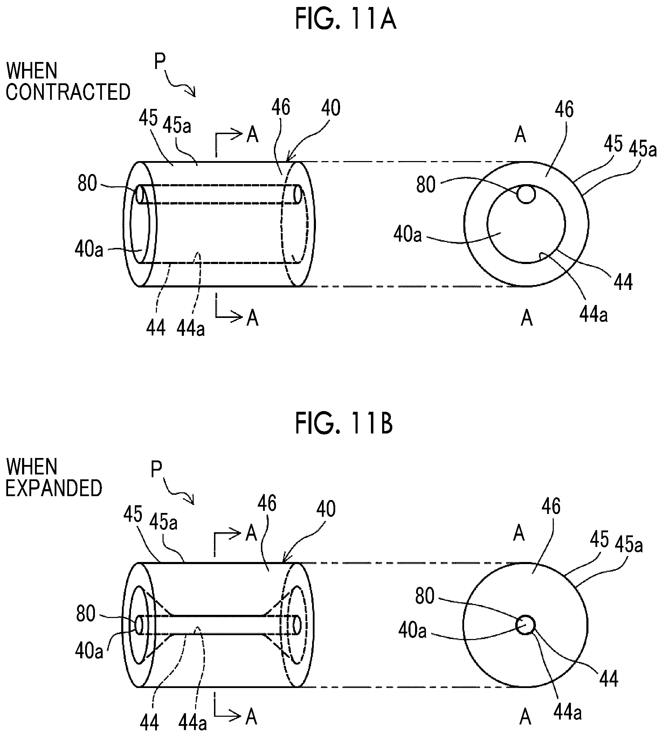

FIG. 8A shows a form in which the spacer 80 is fixed to the center portion of the hollow portion 40a by the fixing portion 81, but as shown in FIG. 11A, the spacer 80 may be fixed to the inner wall surface 44a of the main body portion 40. As shown in FIG. 11A, in a case where the spacer 80 is fixed to the inner wall surface 44a, the spacer 80 is located at substantially the center of the main body portion 40 in a case where the pump portion P is expanded, the inner wall surface 44a is substantially uniformly expanded.

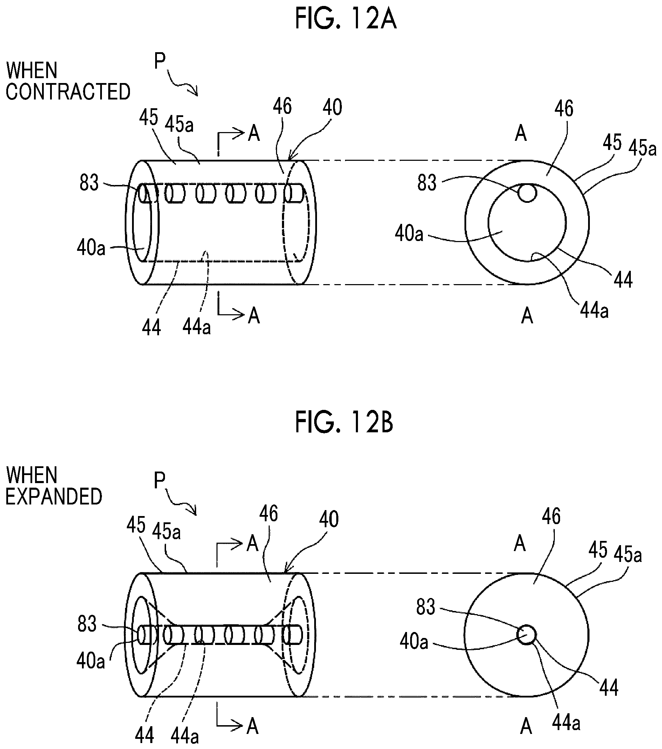

In addition, in the examples shown in FIGS. 8A, 10A, and 11A, the spacers 80 and 82 having a length substantially equal to the length of the main body portion 40 are provided, but as shown in FIGS. 12A and 12B, a plurality of short spacers 83 may be arranged in the axial direction of the main body portion 40. As shown in FIGS. 8A, 10A, and 11A, in a case where only one spacer 80 or 82 is provided, the length of the spacer 80 or 82 is preferably 1/2 to the same as the length of the main body portion 40 in the axial direction, and in a case where the length of the spacer 80 or 82 is shorter than the length of the main body portion 40, the spacer 80 or 82 is preferably disposed at the center portion of the main body portion in the axial direction. In a case where a plurality of short spacers 83 are disposed at intervals in the axial direction of the main body portion 40 as shown in FIGS. 12A and 12B, a total length of the short spacers 83 in the axial direction of the main body portion 40 is preferably 1/2 or more of the length of the main body portion 40 in the axial direction.

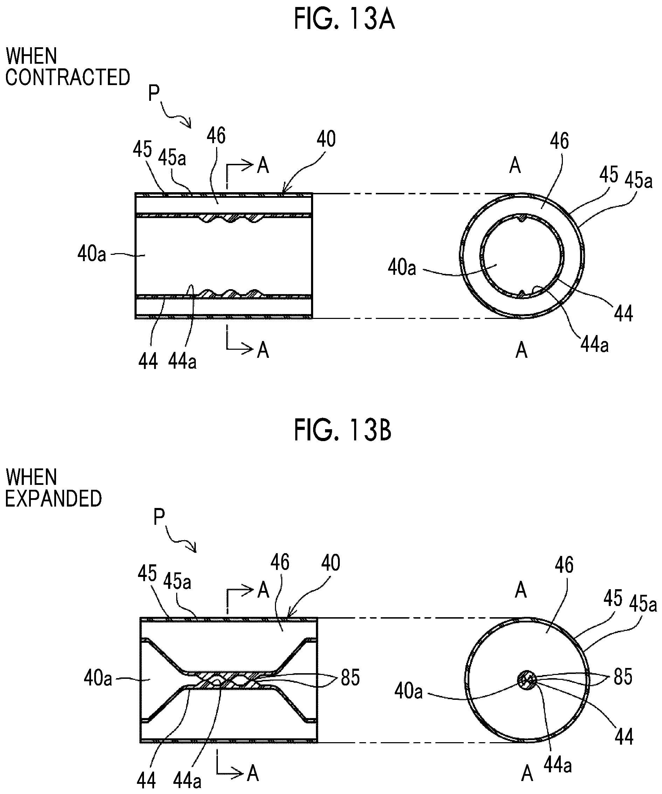

Furthermore, the spacer that prevents the blocking of the hollow portion 40a is not limited to the above-described form. As shown in FIGS. 13A and 13B, a spacer 85 may be provided on the inner wall surface 44a of the inner wall member 44. The spacer 85 is provided as a protrusion that protrudes to the inner diameter side on the inner wall surface 44a as a part of the inner wall member 44. In the present example, a plurality of spacers 85 are provided on the inner wall surface 44a. As shown in FIG. 13B, the spacers 85 disposed at positions facing each other among the spacers 85 are in abutment with each other. Even in a case where the pump portion P is expanded, a space remains in a portion other than a portion in which the spacers 85 are in abutment with each other in the hollow portion 40a, and the hollow portion 40a can be prevented from being completely blocked.

The treatment apparatus 100 of the embodiment described above is configured such that the filtration unit 20 and the bioreactor 10 are connected by one conduit 61. The treatment apparatus according to the present disclosure is not limited to such a configuration, and may include a conduit 63 that is disposed on the second opening 20b side of the filtration unit 20 and that connects the pump portion P and the bioreactor 10 as in the treatment apparatus 101 of the modification example shown in FIG. 14. In a case of the treatment apparatus 101, in a case of the forward feeding in which the culture solution flows from the bioreactor 10 to the filtration unit 20 via the conduit 61, the culture solution that has flowed out from the second opening 20b of the filtration unit 20 is not stopped in the pump portion P, and is fed to the bioreactor 10 via the conduit 63. Then, in a case of the reverse feeding, the culture solution is fed from the bioreactor 10 to the pump portion P on the second opening 20b side of the filtration unit 20 via the conduit 63, and the culture solution flows into the filtration unit 20 through the second opening 20b. It is noted that in the treatment apparatus 101 shown in FIG. 14, the culture solution may be caused to flow through the filtration unit 20 in only the first flow direction or in one direction of the second flow direction without switching the feeding direction.

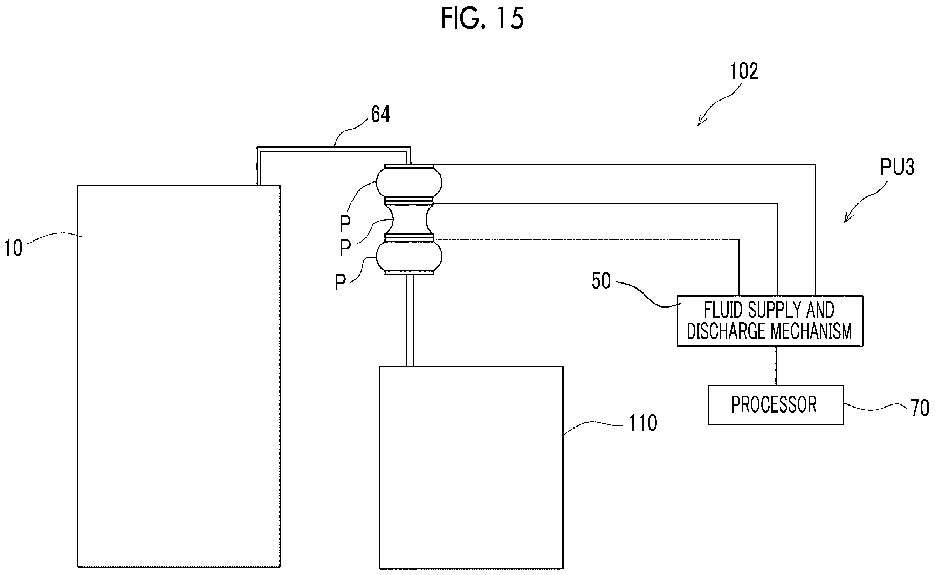

Furthermore, a configuration in which the filtration unit 20 is not provided is also assumed as the treatment apparatus for a cell culture solution according to the present disclosure. FIG. 15 shows a schematic configuration of a treatment apparatus 102 according to another embodiment. The treatment apparatus 102 shown in FIG. 15 includes the bioreactor 10, a conduit 64, a pump unit PU3, a recovery tank 110, and the processor 70. In the present example, the conduit 64, the pump portion P, and the recovery tank 110 constitute a "pipe line in communication with a bioreactor" in the technology of the present disclosure. The pump unit PU3 includes three pump portions P disposed in the pipe line and the fluid supply and discharge mechanism 50 that is a driving unit of the pump portion P. The pump unit PU3 of the present example includes three connected pump portions P, but the number of pump portions P only needs to be two or more.

One end of the conduit 64 is connected to the bioreactor 10, and the other end is connected to the recovery tank 110 via the pump portion P.

The culture solution is fed from the bioreactor 10 to the recovery tank 110 by sequentially expanding and contracting the three pump portions P. In the present example, the processor 70 controls the fluid supply and discharge mechanism 50 to control the timing of the expansion and contraction of each pump portion P to feed the culture solution from the bioreactor 10 to the recovery tank 110.

In this manner, to feed the culture solution from the bioreactor 10 to the recovery tank 110, the treatment apparatus 102 including the pump unit PU3 including two or more pump portions that expand and contract in the radial direction of the pipe line can suppress the damage to the cells as compared with a case where the tube pump in the related art is used. In addition, the flow rate can be increased as compared with a case where the diaphragm pump is used.

In the above-described embodiment, as a hardware structure of the processor 70, various processors shown below can be used. Various processors include a programmable logic device (PLD) that is capable of changing a circuit configuration after manufacturing, such as a field-programmable gate array (FPGA), and a dedicated electric circuit that is a processor having a circuit configuration dedicatedly designed for executing specific processing, such as an application specific integrated circuit (ASIC), in addition to a CPU that is a general-purpose processor configured to execute software (program) to function as various processing units.

The above-described processing may be executed by one of these various processors, or a combination of two or more processors of the same type or different types (for example, a combination of a plurality of FPGAs and a combination of a CPU and an FPGA). In addition, a plurality of processing units may be configured of one processor. As an example, in which the plurality of processing units are configured of one processor, there is a form in which a processor that realizes all functions of a system including the plurality of processing units by using one integrated circuit (IC) chip is used, such as a system on chip (SOC).

Furthermore, the hardware structure of the processors is, more specifically, an electric circuit (circuitry) in which circuit elements such as semiconductor elements are combined.

In addition to the operation program of the analysis apparatus, the technology of the present disclosure extends to a computer readable storage medium (USB memory or digital versatile disc (DVD)-read only memory (ROM), or the like) that stores the operation program of the analysis apparatus in a non-transitory manner.

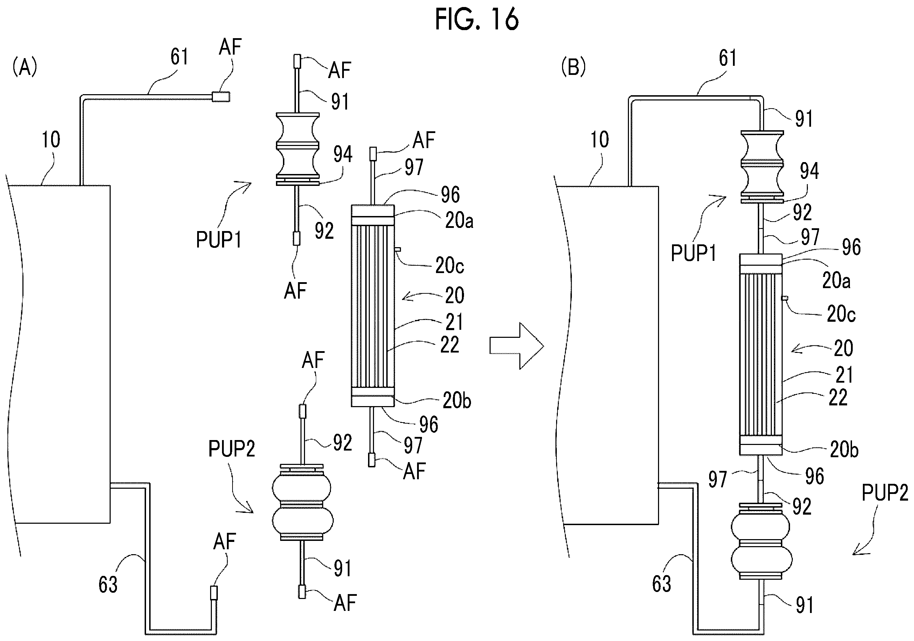

Here, a pipe after the sterilization treatment of the pump unit PU and connection structures with the filtration unit will be described. (A) of FIG. 16 shows a state before the main components of the treatment apparatus 101 shown in FIG. 14 are assembled, and (B) of FIG. 16 shows a state in which the main components are assembled.

As shown in (A) of FIG. 16, an air filter AF is provided at an end portion of a tube constituting the conduit 61 connected to the bioreactor 10. The first pump unit portion PUP1 and the second pump unit portion PUP2 each include tubes 91 and 92 each having an air filter AF at both ends. The pump unit portions PUP1 and PUP2, the tubes 91 and 92, the filtration unit 20, and the tube 97 are connected to each other by using appropriate joints. For example, the first pump unit portion PUP1 and the second pump unit portion PUP2 include a herule 94 on a flange on a side connected to the filtration unit 20, and the tube 92 is connected to the herule 94. Similarly, the filtration unit 20 includes a herule 96 at both ends, and a tube 97 including an air filter AF is connected to each of the herule 96. The air filter AF allows steam to pass therethrough during the steam sterilization, but has a function of preventing the intrusion of foreign substances and the like from the outside. In FIGS. 1 and 14 and the like, a part of the tube and the herule for connecting the units to each other are not shown.

The conduit 61 connected to the bioreactor 10, the first pump unit portion PUP1, the filtration unit 20, and the second pump unit portion PUP2 are subjected to a sterilization treatment such as steam sterilization or gamma-ray sterilization in a state before the assembly shown in (A) of FIG. 16. Then, the tubes constituting the end portions of the units are aseptically connected to each other after the sterilization treatment is performed. Specifically, for example, the tubes are aseptically connected to each other by welding using Biowelder (registered trademark). As shown in (B) of FIG. 16, in a case of the connection, an end portion of the tube including the air filter AF is cut off, and the tubes are connected to each other.

In a case where each unit such as the first pump unit portion PUP1, the second pump unit portion PUP2, and the filtration unit 20 is not used immediately after the sterilization treatment, each unit is stored in a sterilization bag or the like, and is taken out from the sterilization bag and used in a case of use.

It is noted that the first pump unit portion PUP1 and the second pump unit portion PUP2 can be repeatedly used by being washed and subjected to a sterilization treatment after being used in the treatment apparatus 101.

As described above, as an aspect of connecting the conduit 61 connected to the bioreactor 10, the first pump unit portion PUP1, the filtration unit 20, and the second pump unit portion PUP2 as shown in (A) of FIG. 16, in addition to an aspect of aseptically connecting the tubes to each other, an aspect of providing a connector for aseptic connection at an end portion of the tube and connecting the connectors to each other may be used. Examples of the connector for aseptic connection include a CPC coupling for aseptic connection of Colder Products Company (CPC).

The contents described and shown above are detailed descriptions of portions according to the technology of the present disclosure and are merely examples of the technology of the present disclosure. For example, the above description of the configurations, functions, operations, and effects is the description of examples of the configurations, functions, operations, and effects of portions related to the technology of the present disclosure. Therefore, unnecessary portions may be deleted or new elements may be added or replaced in the above descriptions and illustrations without departing from the gist of the technology of the present disclosure. In addition, to avoid complication and facilitate understanding of portions according to the technology of the present disclosure, description related to common technical knowledge or the like that does not need to be particularly described for enabling implementation of the technology of the present disclosure is omitted in the contents described and shown above.

The contents of the disclosures of JP2023-166525A filed on September 27, 2023, and JP2024-023031 filed on February 19, 2024, are incorporated in the present specification by reference in their entirety.

All documents, patent applications, and technical standards disclosed in the present specification are incorporated in the present specification by reference to the same extent as those in a case where each of the documents, patent applications, and technical standards are specifically and individually indicated to be incorporated by reference.

In regard to the above-described embodiment, following appendixes are further disclosed.

### <Appendix 1>

A treatment apparatus for a cell culture solution, the treatment apparatus comprising:
a bioreactor that accommodates the cell culture solution;
a pipe line in communication with the bioreactor; and
a pump unit that feeds the cell culture solution to the pipe line,
wherein the pump unit includes two or more pump portions that are disposed in the pipe line and that expand and contract in a radial direction of the pipe line.

### <Appendix 2>

The treatment apparatus for a cell culture solution according to appendix 1,
wherein the pump portion includes a tubular member that has a hollow portion through which the cell culture solution flows, that has a space between an inner wall surface and an outer wall surface, and that has elasticity such that at least the inner wall surface expands and contracts in the radial direction of the pipe line, and
the pump unit includes a fluid supply and discharge mechanism that expands the pump portion by supplying a fluid into the space between the inner wall surface and the outer wall surface of the tubular member, and that contracts the pump portion by discharging the fluid from the space.

### <Appendix 3>

The treatment apparatus for a cell culture solution according to appendix 1,
wherein the pump portion is a tubular member that has a hollow portion through which the cell culture solution flows, that has a space between an inner wall surface and an outer wall surface, and that has elasticity such that at least the inner wall surface expands and contracts in the radial direction of the pipe line, and
the pump unit includes a pressing-force applying mechanism that expands the pump portion by mechanically pressing the inner wall surface of the tubular member toward a radially inner side of the pipe line, and that contracts the pump portion by releasing the pressing, the pump portion being contracted by an elastic force of the tubular member.

### <Appendix 4>

The treatment apparatus for a cell culture solution according to any one of appendixes 1 to 3,
wherein a ratio of cross-sectional areas of a flow path of the pump portion in a case where the pump portion is expanded and the flow path of the pump portion in a case where the pump portion is contracted is 0.01 or more.

### <Appendix 5>

The treatment apparatus for a cell culture solution according to any one of appendixes 1 to 4,
wherein a spacer that prevents a flow path of the pump portion from being blocked in a case where the pump portion is expanded is disposed in the flow path of the pump portion.

### <Appendix 6>

The treatment apparatus for a cell culture solution according to appendix 5,
wherein the spacer is a tubular member formed of a mesh material and having an outer diameter smaller than an inner diameter of the flow path.

### <Appendix 7>

The treatment apparatus for a cell culture solution according to appendix 5,
wherein the spacer is provided on a wall surface of the flow path of the pump portion.

### <Appendix 8>

The treatment apparatus for a cell culture solution according to any one of appendixes 1 to 7, further comprising:
a filtration unit through which the cell culture solution flows in and out and that is disposed in the pipe line and includes a discharge port through which the cell culture solution flows in and out and a filtrate is discharged,
wherein the cell culture solution is fed from the bioreactor to the filtration unit by expansion and contraction of the pump portions.

### <Appendix 9>

The treatment apparatus for a cell culture solution according to appendix 8,
wherein, in the pipe line, the pump portions are disposed on each of a downstream side and an upstream side of the filtration unit.

### <Appendix 10>

The treatment apparatus for a cell culture solution according to appendix 8 or 9,
wherein, in the pipe line, the filtration unit is connected to the bioreactor by one conduit.

### <Appendix 11>

The treatment apparatus for a cell culture solution according to any one of appendixes 1 to 10, further comprising:
a recovery tank that is disposed in the pipe line and recovers the cell culture solution,
wherein the cell culture solution is fed from the bioreactor to the recovery tank by expansion and contraction of the pump portions.

### <Appendix 12>

A method for treating a cell culture solution, the method comprising:
a feeding step of disposing, in a pipe line in communication with a bioreactor that accommodates the cell culture solution, two or more pump portions that expand and contract in a radial direction of the pipe line, and feeding the cell culture solution by expanding and contracting the pump portions.

### <Appendix 13>

The method for treating a cell culture solution according to appendix 12,
wherein a filtration unit through which the cell culture solution flows in and out and that includes a discharge port through which a filtrate is discharged, is further provided in the pipe line,
the feeding step is a step of feeding the cell culture solution from the bioreactor to the filtration unit, and
the method includes a step of collecting the filtrate from the filtration unit after the feeding step.

### <Appendix 14>

The method for treating a cell culture solution according to appendix 13,
wherein the feeding step includes:
(a) a step of causing the cell culture solution to flow from the bioreactor through the filtration unit for a first period in a first flow direction by sequentially expanding and contracting the two or more pump portions;
(b) a step of causing the cell culture solution to flow through the filtration unit for a second period in a second flow direction in which the first flow direction is reversed by sequentially expanding and contracting the two or more pump portions in an order opposite to that in the step (a);
(c) a step of causing the cell culture solution to flow through the filtration unit for a third period in the first flow direction in which the second flow direction is reversed by sequentially expanding and contracting the two or more pump portions in the same order as that in the step (a); and
(d) a step of repeating the steps (b) and (c) at least twice.

### <Appendix 15>

The method for treating a cell culture solution according to appendix 14,
wherein the first period and the second period are each 30 seconds or less.

### <Appendix 16>

A method for producing a product which is a product obtained by culturing cells, the method comprising:
a culturing step of culturing the cells in a bioreactor that accommodates a cell culture solution;
a feeding step of disposing, in a pipe line in communication with the bioreactor, two or more pump portions that expand and contract in a radial direction of the pipe line, and feeding the cell culture solution to a filtration unit disposed on the pipe line by expanding and contracting the pump portions; and
a collection step of collecting a filtrate containing the product filtered from the cell culture solution by the filtration unit.

### <Appendix 17>

An apparatus for producing a product, the apparatus comprising:
a bioreactor that accommodates a cell culture solution;
a pipe line in communication with the bioreactor;
a filtration unit that is disposed in the pipe line and filters the cell culture solution to obtain a filtrate containing the product; and
a pump unit that feeds the cell culture solution from the bioreactor to the filtration unit,
wherein the pump unit includes two or more pump portions that are disposed in the pipe line and that expand and contract in a radial direction of the pipe line.

## Claims

1. A treatment apparatus for a cell culture solution, the treatment apparatus comprising:
a bioreactor that accommodates the cell culture solution;
a pipe line in communication with the bioreactor; and
a pump unit that feeds the cell culture solution to the pipe line,
wherein the pump unit includes two or more pump portions that are disposed in the pipe line and that expand and contract in a radial direction of the pipe line.

2. The treatment apparatus for a cell culture solution according to claim 1,
wherein the pump portion includes a tubular member that has a hollow portion through which the cell culture solution flows, that has a space between an inner wall surface and an outer wall surface, and that has elasticity such that at least the inner wall surface expands and contracts in the radial direction of the pipe line, and
the pump unit includes a fluid supply and discharge mechanism that expands the pump portion by supplying a fluid into the space between the inner wall surface and the outer wall surface of the tubular member, and that contracts the pump portion by discharging the fluid from the space.

3. The treatment apparatus for a cell culture solution according to claim 1,
wherein the pump portion is a tubular member that has a hollow portion through which the cell culture solution flows, that has a space between an inner wall surface and an outer wall surface, and that has elasticity such that at least the inner wall surface expands and contracts in the radial direction of the pipe line, and
the pump unit includes a pressing-force applying mechanism that expands the pump portion by mechanically pressing the inner wall surface of the tubular member toward a radially inner side of the pipe line, and that contracts the pump portion by releasing the pressing, the pump portion being contracted by an elastic force of the tubular member.

4. The treatment apparatus for a cell culture solution according to claim 1,
wherein a ratio of cross-sectional areas of a flow path of the pump portion in a case where the pump portion is expanded and the flow path of the pump portion in a case where the pump portion is contracted is 0.01 or more.

5. The treatment apparatus for a cell culture solution according to claim 1,
wherein a spacer that prevents a flow path of the pump portion from being blocked in a case where the pump portion is expanded is disposed in the flow path of the pump portion.

6. The treatment apparatus for a cell culture solution according to claim 5,
wherein the spacer is a tubular member formed of a mesh material and having an outer diameter smaller than an inner diameter of the flow path.

7. The treatment apparatus for a cell culture solution according to claim 5,
wherein the spacer is provided on a wall surface of the flow path of the pump portion.

8. The treatment apparatus for a cell culture solution according to any one of claims 1 to 7, further comprising:
a filtration unit through which the cell culture solution flows in and out and that is disposed in the pipe line and includes a discharge port through which the cell culture solution flows in and out and a filtrate is discharged,
wherein the cell culture solution is fed from the bioreactor to the filtration unit by expansion and contraction of the pump portions.

9. The treatment apparatus for a cell culture solution according to claim 8,
wherein, in the pipe line, the pump portions are disposed on each of a downstream side and an upstream side of the filtration unit.

10. The treatment apparatus for a cell culture solution according to claim 8,
wherein, in the pipe line, the filtration unit is connected to the bioreactor by one conduit.

11. The treatment apparatus for a cell culture solution according to any one of claims 1 to 7, further comprising:
a recovery tank that is disposed in the pipe line and recovers the cell culture solution,
wherein the cell culture solution is fed from the bioreactor to the recovery tank by expansion and contraction of the pump portions.

12. A method for treating a cell culture solution, the method comprising:
a feeding step of disposing, in a pipe line in communication with a bioreactor that accommodates the cell culture solution, two or more pump portions that expand and contract in a radial direction of the pipe line, and feeding the cell culture solution by expanding and contracting the pump portions.

13. The method for treating a cell culture solution according to claim 12,
wherein a filtration unit through which the cell culture solution flows in and out and that includes a discharge port through which a filtrate is discharged, is further provided in the pipe line,
the feeding step is a step of feeding the cell culture solution from the bioreactor to the filtration unit, and
the method includes a step of collecting the filtrate from the filtration unit after the feeding step.

14. The method for treating a cell culture solution according to claim 13,
wherein the feeding step includes:
(a) a step of causing the cell culture solution to flow from the bioreactor through the filtration unit for a first period in a first flow direction by sequentially expanding and contracting the two or more pump portions;
(b) a step of causing the cell culture solution to flow through the filtration unit for a second period in a second flow direction in which the first flow direction is reversed by sequentially expanding and contracting the two or more pump portions in an order opposite to that in the step (a);
(c) a step of causing the cell culture solution to flow through the filtration unit for a third period in the first flow direction in which the second flow direction is reversed by sequentially expanding and contracting the two or more pump portions in the same order as that in the step (a); and
(d) a step of repeating the steps (b) and (c) at least twice.

15. The method for treating a cell culture solution according to claim 14,
wherein the first period and the second period are each 30 seconds or less.

16. A method for producing a product which is a product obtained by culturing cells, the method comprising:
a culturing step of culturing the cells in a bioreactor that accommodates a cell culture solution;
a feeding step of disposing, in a pipe line in communication with the bioreactor, two or more pump portions that expand and contract in a radial direction of the pipe line, and feeding the cell culture solution to a filtration unit disposed on the pipe line by expanding and contracting the pump portions; and
a collection step of collecting a filtrate containing the product filtered from the cell culture solution by the filtration unit.
